# EUROPEAN PATENT SPECIFICATION

(11) **EP 4 019 024 B1**
(45) Date of publication and mention of the grant of the patent: **19.02.2025**
(21) Application number: 20859608.0
(22) Date of filing: 19.08.2020
(51) Int. Cl.: A61K 31/739, A23L 33/125, A61K 31/7008, A61K 35/741, A61P 35/00, A61P 43/00, A61K 31/513, A61K 31/675, A61K 31/7088, A61K 33/243, A61K 45/06

(54) **CANCER CHEMOTHERAPY SUPPORTING AGENT, FOOD, AND DRUG**
KREBSCHEMOTHERAPIE-UNTERSTÜTZENDES MITTEL, NAHRUNGSMITTEL UND ARZNEIMITTEL
AGENT ASSISTANT UNE CHIMIOTHÉRAPIE ANTICANCÉREUSE, ALIMENT ET MÉDICAMENT

(30) Priority: 29.08.2019 JP 2019156220
(43) Date of publication of application: 29.06.2022
(73) Proprietor: Biomedical Research Group Inc., Tokyo 158-0084 (JP); SOMA Gen-Ichiro, Setagaya-ku Tokyo 158-0084 (JP)
(72) Inventor: MIZOBUCHI Haruka, Takamatsu-shi, Kagawa 761-0301 (JP); INAGAWA Hiroyuki, Takamatsu-shi, Kagawa 761-8062 (JP); KOHCHI Chie, Takamatsu-shi, Kagawa 761-8075 (JP); SOMA Gen-Ichiro, Tokyo 158-0084 (JP)
(74) Representative: Appelt, Christian W.
(86) International application number: PCT/JP2020/031357
(87) International publication number: WO 2021/039554

(56) References cited:
- WO-A1-2012/173163
- WO-A1-91/14424
- JP-A- 2012 082 156
- JP-A- 2013 136 787
- KOHCHI C ET AL: "Applications of lipopolysaccharide derived from Pantoea agglomerans (IP-PA1) for health care based on macrophage network theory", JOURNAL OF BIOSCIENCE AND BIOENGINEERING, ELSEVIER, AMSTERDAM, NL, vol. 102, no. 6, 1 December 2006 (2006-12-01), pages 485 - 496, XP028042366, ISSN: 1389-1723, [retrieved on 20061201], DOI: 10.1263/JBB.102.485
- HULL M.A ET AL: "Activity of the non-steroidal anti-inflammatory drug indomethacin against colorectal cancer", CANCER TREATMENT REVIEWS, vol. 29, no. 4, 1 August 2003 (2003-08-01), AMSTERDAM, NL, pages 309 - 320, XP093135092, ISSN: 0305-7372, DOI: 10.1016/S0305-7372(03)00014-8
- TAKEHISA HEBISHIMA ET AL: "Protective Effects of the Immunopotentiator from Pantoea agglomerans 1 on Chemotherapeutic Agent-induced Macrophage Growth Inhibition", ANTICANCER RESEARCH, vol. 30, no. 6, 1 June 2010 (2010-06-01), pages 2033 - 2040, XP055954680
- HEBISHIMA TAKEHISA ET AL: "Oral Administration of Immunopotentiator from Pantoea agglomerans 1 (IP-PA1) Improves the Survival of B16 Melanoma-Inoculated Model Mice", EXPERIMENTAL ANIMALS, vol. 60, no. 2, 1 January 2011 (2011-01-01), JP, pages 101 - 109, XP055797836, ISSN: 1341-1357, Retrieved from the Internet <URL:https://www.jstage.jst.go.jp/article/expanim/60/2/60_2_101/_pdf/-char/en> DOI: 10.1538/expanim.60.101
- DUTKIEWICZ JACEK ET AL: "Pantoea agglomerans: a mysterious bacterium of evil and good. Part IV. Beneficial effects", ANNALS OF AGRICULTURAL AND ENVIRONMENTAL MEDICINE, vol. 23, no. 2, 2 June 2016 (2016-06-02), PL, pages 206 - 222, XP055793240, ISSN: 1232-1966, DOI: 10.5604/12321966.1203879
- MARECKI N M ET AL: "Effect of bacterial lipopolysaccharide, lipid A, and concanavalin a on lethality of 5-fluorouracil for mice", TOXICOLOGY AND APPLIED PHARMACOLOGY, ACADEMIC PRESS, AMSTERDAM, NL, vol. 31, no. 1, 1 January 1975 (1975-01-01), pages 83 - 92, XP024882390, ISSN: 0041-008X, [retrieved on 19750101], DOI: 10.1016/0041-008X(75)90054-X
- ATSUTOMO MORISHIMA ET AL: "Clinical Effects of Orally Administered Lipopolysaccharide Derived from Pantoea agglomerans on Malignant Tumors", ANTICANCER RESEARCH, vol. 36, no. 7, 1 July 2016 (2016-07-01), pages 3747 - 3752, XP055954655
- HEBISHIMA TAKEHISA, MATSUMOTO YASUNOBU, WATANABE GEN, SOMA GEN-ICHIRO, KOHCHI CHIE, TAYA KAZUYOSHI, HAYASHI YOSHIHIRO, HIROTA YOSH: "Oral Administration of Immunopotentiator from Pantoea agglomerans 1 (IP- PA1) Improves the Survival of B16 Melanoma- Inoculated Model Mice", EXP. ANIM., vol. 60, no. 2, 2011, pages 101 - 109, XP055797836
- INAGAWA, HIROYUKI: "The beginning of natural immunity to eat. 5. LPS is a functional food ingredient", FOOD STYLE 21, vol. 18, no. 1, 30 November 2013 (2013-11-30), JP , pages 41 - 43, XP009534968, ISSN: 1343-9502
- CHIBA, YUKO ET AL.: "Antitumor effect of low molecular Lipopolysaccharide (LPS) with percutaneous administration", BIOTHERAPY, vol. 7, no. 3-1, 30 November 1992 (1992-11-30), JP , pages 352 - 353, XP009534980, ISSN: 0914-2223
- SOMA, GENICHIRO: "Physiological Significance of LPS (Lipopolysaccharide)", BULLETIN OF KAGAWA JUNIOR COLLEGE, vol. 45, 30 November 2016 (2016-11-30), JP, pages 333 - 349, XP009534986, ISSN: 0387-3692

## Description

### [Technical Field]

The present invention relates to a cancer chemotherapy supporting agent, food, and drug for reducing side effects of cancer chemotherapeutic agents.

### [Background Art]

### <Statistics related to cancer>

According to statistics of the Ministry of Health, Labour and Welfare, cancer was the No. 1 cause of death in Japan in 2017 and constituted 27.9% of all causes of death (NPL 1). According to statistics compiled by the National Cancer Center Japan, the risk of contracting cancer during a lifetime is 62% for males and 47% for females, the lifetime cancer mortality risk is 25% for males and 15% for females, and cancer is the top cause of death of Japanese (NPL 2).

### <Choices of cancer treatment>

Although treatment methods for cancer differ greatly according to site and type, generally, there are the choices of operative therapy, chemotherapy, radiation therapy, and immunotherapy, and there are also many cases where these are combined. Among these, chemotherapy (anticancer drug treatment) is the most standard therapy used and also used in combination with other therapies.

Cancer chemotherapeutic agents include
- antimetabolites (substances that inhibit use of metabolites that are produced and exhibit DNA synthesis inhibitory action on dividing cells; drug examples: 5-fluorouracil (5-FU), cytarabine, cladribine, etc.),
- alkylating agents (exhibit cytostatic action by alkylating DNA and crosslinking guanine bases of the double chain to each other; drug examples: cyclophosphamide, melphalan, ifosfamide, busulfan, streptozotocin, etc.),
- microtubule affecting agents (exhibit polymerization inhibiting action by binding to tubulin that forms microtubules; drug examples: paclitaxel, docetaxel, vincristine, vinblastine, etc.),
- topoisomerase inhibiting agents (exhibit action of inhibiting DNA rebinding by acting on topoisomerase that synthesizes the DNA of cancer cells; drug examples: irinotecan, etoposide, etc.),
- anticancer antibacterial agents (action mechanism differs according to drug; drug examples: doxorubicin, bleomycin, mitomycin C, etc.),
- platinating agents (exhibit DNA insertion and polymerase inhibiting actions; drug examples: cisplatin, epirubicin, carboplatin, etc.)
(NPL 3). For all of these, the grounds of selectivity are deemed to be based on cancer cells being higher in proliferative ability than normal cells.

### <Side effects of chemotherapeutic agents>

However, with chemotherapeutic agents, even normal cells that are high in mitotic rate such as blood (bone marrow) cells, mucosal tissue cells, hair root cells, etc., receive inhibitory actions like cancer cells. Consequently, increased susceptibility to infection, anemia, bleeding, nausea, stomatitis, diarrhea, change in taste sensation, hair loss, skin disorder, change in nails, death, and other symptoms occur as side effects (NPL 4). Examples of chemotherapeutic agents with which the frequency of side effects is high and nausea occurs readily are AC therapy (doxorubicin + cyclophosphamide), EC therapy (epirubicin + cyclophosphamide), cyclophosphamide, cisplatin, streptozocin, dacarbazine, etc., and these have an emesis incidence exceeding 90%. Also, actinomycin D, azacytidine, amrubicin, idarubicin, ifosfamide, irinotecan, enocitabine, epirubicin, oxaliplatin, carboplatin, clofarabine, arsenic trioxide, cyclophosphamide, cytarabine, daunorubicin, temozolomide, doxorubicin, nedaplatin, pirarubicin, busulfan, bendamustine, miriplatin, methotrexate, melphalan, etc., are also deemed to have an emesis incidence of 30 to 90%. As antiemetics for emesis, aprepitant, serotonin receptor antagonists, and dexamethasone are mainly used. Further, late onset diarrhea associated with intestinal mucosal disorder occurs frequently as a side effect of anticancer agents. Irinotecan, methotrexate, docetaxel, 5-FU, capecitabine, cytarabine, doxorubicin, etc., are known as cancer chemotherapeutic agents with which diarrhea is frequently observed. With adriamycin, epirubicin, docetaxel, and paclitaxel, the hair of the head falls off in most patients. Due to these side effects, many patients give up on chemotherapy in the middle of treatment. Also, infection during leucopenia is deemed to be the most frequent cause of death as side effect of anticancer agents (NPL 5).

### <Conventional arts for reducing side effects>

Side effects occur in most chemotherapeutic agent treatments and in some cases, the side effect emerges more strongly than the effect (NPL 3). Such a background gave rise in recent years to the concept of symptomatic therapy (supportive therapy) for relieving symptoms with respect to symptoms accompanying cancer and side effects due to treatment. Specifically, drugs for suppressing nausea (serotonin blockers and dexamethasone), antidiarrheics and intestinal regulators for diarrhea, purgatives for constipation, anti-inflammatory agents and analgesics for stomatitis, antibiotics for infection, blood transfusion for supplementing anemia, vitamin preparations for numbness caused by microtubule affecting agents etc., are used (NPL 6) . However, although there are patients with whom alleviation of symptoms to some degree is seen, sufficient effects are not obtained in many cases and it cannot yet be said that an effective supportive therapy is established (NPL 7).

Current supporting therapies are symptomatic treatment agents administered to patients after symptoms are expressed as side effects due to chemotherapeutic agents in accordance with the symptoms. If side effects of chemotherapeutic agents can be reduced with foods, this would be a safe and excellent supportive therapy.

Hebishima et al. [NPL 12]disclose that IP-PA1, i.e. the edible lipopolysaccharide (LPS) derived from the enterobacter *Pantoea agglomerans 1,* is an immunopotentiator with protective effects on chemotherapeutic agent-induced macrophage growth inhibition.

Although Kampc medicines that reduce side effects of cancer chemotherapeutic agents have been reported up to now (NPL 7), safe foods or food ingredients that patients can ingest freely have not been established. There has thus been a demand for safe foods, food ingredients (supplements), and drugs that can safely prevent side effects of chemotherapeutic agents.

### [Citation List]

### [Non Patent Literature]

[NPL 1] "Numbers of Deaths, Death Rates (per 100,000 population), and Proportions of 10 Leading Causes of Death by Sex.", [online], Sept. 7, 2018, Ministry of Health, Labour and Welfare, [retrieved on Aug. 21, 2019], Internet, <https://www.mhlw.go.jp/toukei/saikin/hw/jinkou/kakutei17/ dl/10_h6.pdf>
[NPL 2] "Latest Cancer Statistics", [online], Jan. 21, 2019, National Cancer Center Japan, [retrieved on Aug. 21, 2019], Internet, <https://ganjoho.jp/reg_stat/statistics/stat/summary.html>
[NPL 3] "Cytotoxic Anticancer Agents: Controlling Side Effects by Combined Use of Preventive Drugs and Treatment Drugs", [online], June 28, 2011, AKIRAMENAI-GANCHIRYO-NETWORK, [retrieved on Aug. 21, 2019], Internet, <https://www.akiramenai-gan.com/medical_contents/disease/1 927/2/>
[NPL 4] "Side Effects of Chemotherapy", [online], Feb. 2, 2016, National Cancer Center Japan, [retrieved on Aug. 21, 2019], Internet, <https://ganjoho.jp/child/dia_tre/treatment/side_effect01. html>
[NPL 5] "Misunderstandings about Anticancer Agents and Side Effects - Can One Eat Raw Food During Anticancer Agent Treatment? -", [online], Dec. 14, 2015, The Yomiuri Shimbun, [retrieved on Aug. 21, 2019], Internet, <https://yomidr.yomiuri.co.jp/article/20151214-OYTEW55388/>
[NPL 6] "Pharmacotherapy (Chemotherapy)", [online], July 22, 2019, National Cancer Center Japan, [retrieved on Aug. 21, 2019], Internet, <https://ganjoho.jp/public/dia_tre/treatment/drug_therapy. html>
[NPL 7] Naohisa Yoshida and 2 others, "<Special Issue: Latest Information on Kampo Therapy.> Efficacy of Kampo Medicine for Side Effect of Anti-cancer Drug Therapy", Journal of Kyoto Prefectural University of Medicine, 2016, 125(2), pp. 115-125.
[NPL 8] H. Inagawa et al., "Primed Activation of Macrophages by Oral Administration of Lipopolysaccharide Derived from Pantoea agglomerans", in vivo, 2016, 30, pp. 205-211.
[NPL 9] Y. Kobayashi et al., "Oral administration of Pantoea agglomerans-derived lipopolysaccharide prevents metabolic dysfunction and Alzheimer's disease-related memory loss in senescence-accelerated prone 8 (SAMP8) mice fed a high-fat diet", PLoS ONE. 2018, 13(6): e0198493.
[NPL 10] Wikipedia Japan "Streptozotocin", [retrieved on Aug. 21, 2019], Internet
[NPL 11] T. Hebishima et al., "Oral Administration of Immunopotentiator from Pantoea agglomerans 1 (IP-PA1) Improves the Survival of B16 Melanoma-Inoculated Model Mice", Exp. Anim., 2011, 60(2), pp. 101-109.
[NPL 12] T. Hebishima et al.: "Protective Effects of the Immunopotentiator from Pantoea agglomerans 1 on Chemotherapeutic Agent-induced Macrophage Growth Inhibition", Anticancer Research, 2010, 30(6), pp. 2033 - 2040.

### [Summary of Invention]

### [Technical Problem]

In view of the current circumstances described above, an object of the present invention is to provide a safe food-derived ingredient that reduces side effects of a cancer chemotherapeutic agent. In particular, an object is to provide a food, a food ingredient (supplement), and a drug that reduce lethal toxic effects of a chemotherapeutic agent.

### [Solution to Problem]

A cancer chemotherapy supporting agent, food, or drug according to the present invention is a cancer chemotherapy supporting agent, food, or drug for reducing side effects of cancer chemotherapy and increasing the administration amounts of chemotherapeutic agents that is characterized in containing a lipopolysaccharide as an active ingredient, wherein the supporting agent is orally administered, and
- wherein the cancer chemotherapy is that in which an alkylating agent is administered, wherein the alkylating agent is streptozotocin, and the side effect is a decreased survival rate induced by streptozotocin; or
- wherein the cancer chemotherapy is that in which a platinating agent is administered, wherein the platinating agent is cisplatin, and the side effect is a decreased survival rate induced by cisplatin; or
- wherein the cancer chemotherapy is that in which an alkylating agent is administered, wherein the alkylating agent is cyclophosphamide, and the side effect is weight loss induced by cyclophosphamide; or
- wherein the cancer chemotherapy is that in which an antimetabolite is administered, wherein the antimetabolite is 5-fluorouracil, and the side effect is weight loss induced by 5-fluorouracil.

According to a further embodiment, the lipopolysaccharide is derived from a bacterium belonging to family Enterobacteriaceae.

According to a further embodiment, the lipopolysaccharide is derived from a bacterium belonging to genus Pantoea or derived from a bacterium belonging to genus Enterobacter.

To achieve the above object, the present inventors found from research results up to now that by allowing oral ingestion of a lipopolysaccharide (LPS) derived from gram negative bacteria and having a function of increasing healing power, a novel supportive therapy based on ingestion of a safe food, food ingredient (supplement), or drug that reduces side effects of a chemotherapeutic agent can be constructed.

A living body, upon receiving cellular damage or tissue damage, is cured by induction of cytokines, involved in cellular proliferation and tissue repair from phagocytes (macrophages etc.) and epithelial cells, such as fibroblast growth factors (FGF) vascular endothelial growth factor (VEGF), keratinocyte growth factor (KGF), platelet-derived growth factors (PDGF), tumor necrosis factor (TNF), etc., and interleukin-6 (IL-6) etc. LPSs are also known to induce these cytokines by binding to toll-like receptor 4 (TLR4) expressed on phagocytes and epithelial cells in cell assays or by injection administration. However, LPS oral administration differs completely in effect from cell stimulation assays and an effect of inducing the above cytokines that promote recovery from damage of a living body by oral administration of LPSs was not known (NPL 8).

When the influence of LPS oral administration on glial cell line-derived neurotrophic factor (GNDF), which is known to exhibit cell death inhibition, was examined, it was found that gene expression of glial cell line-derived neurotrophic factor (GNDF) is increased significantly by LPS oral administration. This suggests the possibility that LPS oral administration exhibits an action of preventing cellular damage or tissue damage of a living body that is caused by one reason or another.

A cancer chemotherapeutic agent inflicts damage on normal dividing cells and there is thus a possibility that damage is occurring even in tissue with which a side effect is not apparent. In tissue in which cellular damage occurs concentratedly, this is observed as a symptom of side effect in the tissue. The most serious side effect is death of an individual. It is thus considered that the benefits of oral ingestion of LPS as a supportive therapy can be evaluated using an animal model in which death of an individual induced by cancer chemotherapy is prevented. With the present invention, death prevention effect by oral administration of LPS was clarified using models of death by cancer chemotherapeutic agents in mice.

The LPSs that are used as supportive therapy agents are substances that are present in the environment and are substances that are not only ingested to some degree even in daily life but are also being blended in foods and cosmetics. Through oral ingestion, LPSs increase functionality of intraperitoneal and intracerebral phagocytes (macrophages) (NPLs 8 and 9). Also, activated phagocytes have a function of repairing tissue damage inside a living body. Although as LPSs, LPSs derived from bacteria belonging to genus Pantoea, bacteria belonging to genus Enterobacter, bacteria belonging to genus Xanthomonas, bacteria belonging to family Acetobacteraceae, and bacteria belonging to genus Zymomonas that are plant symbiotic bacteria with history of use in foods can be cited, as long as an LPS is used, it is not necessary to specify the bacterial strain.

As cancer chemotherapeutic agents, generally used antimetabolites (substances that inhibit use of metabolites that are produced and inhibit DNA production in dividing cells; **5-fluorouracil** (5-FU), cytarabine, cladribine, etc.), alkylating agents (that stop cell division by alkylating DNA and crosslinking guanine bases of the double chain to each other; cyclophosphamide, melphalan, ifosfamide, busulfan, streptozotocin, etc.), microtubule affecting agents (that bind to tubulin, which forms microtubules, to inhibit polymerization; paclitaxel, docetaxel, vincristine, vinblastine, etc.), topoisomerase inhibiting agents (that inhibit rebinding of DNA by acting on topoisomerase that synthesizes the DNA of cancer cells; irinotecan, etoposide, etc.), anticancer antibacterial agents (action mechanism differs according to drug; doxorubicin, bleomycin, mitomycin C, etc.), platinating agents (that inhibit DNA insertion and polymerases; cisplatin, epirubicin, carboplatin, etc.) and the like that exhibit cytotoxic action are applicable.

The present invention is directed to the reduction of certain specific side effects, induced by certain specific chemotherapeutic agents, as defined in the appended claims.

### [Advantageous Effects of Invention]

By side effects (increased susceptibility to infection, anemia, bleeding, nausea, stomatitis, diarrhea, change in taste sensation, hair loss, skin disorder, change in nails, death, etc., not all covered by the present invention) due to cancer chemotherapeutic agents being reduced by oral ingestion of the LPS of the present invention, patients being treated for cancer will become able to lead an ordinary life without degradation of quality of life (QOL). Also, by making it possible to increase the administration amounts of chemotherapeutic agents, increase in cancer curing effect can also be anticipated.

LPSs are food ingredients and since there are no side effects at all even when ingested by a healthy individual, foods and food ingredients (supplements) can be ingested by anybody. The advantages that use in combination with various choices of cancer treatment is possible and that continued use before, during, and even after treatment does not present a problem are thus provided.

### [Brief Description of Drawings]

[FIG. 1] is a diagram showing results of an experiment of Example 1 of the present invention.
[FIG. 2] is a diagram showing results of an experiment of Example 2 of the present invention.
[FIG. 3] is a diagram showing results of an experiment of Example 5 of the present invention.

### [Description of Embodiments]

Examples of the present invention shall be described.

### [Example 1]

Reduction of toxic effects of the chemotherapeutic agent streptozotocin by LPS oral administration
(1) Method
   1) Reagents
      - LPS (LPSp) derived from Pantoea agglomerans belonging to genus Pantoea of family Enterobacteriaceae (mac0001 of Macrophi Inc.)
      - Streptozotocin (STZ) (SAJ-S0130 of Sigma-Aldrich)
   2) Mice and husbandry
      Six-week-old male C57BL mice were used. Husbandry of the mice was carried out in a temperature and humidity controlled animal facility under environmental conditions of ad libitum food and water, and 12 hr light/12 hr dark. As food, the D12450B diet of Research Diets, Inc. was provided. After 1 week of preliminary rearing, the mice were divided into 3 groups of 6 mice each.
   3) Test

PBS administration group (PBS i.p.): 0.5 mL of phosphate buffer saline (PBS) were injected into the peritoneal cavity of each mouse.

STZ administration group (STZ i.p.) : An STZ solution (250 mg/kg) was injected into the peritoneal cavity of each mouse.

STZ + LPS administration group (STZ i.p.+LPS): The STZ solution (250 mg/kg) was injected into the peritoneal cavity of each mouse and LPSp was mixed in drinking water such as to be of 100 µg/kg body weight/day and allowed to be ingested ad libitum.

### (2) Results

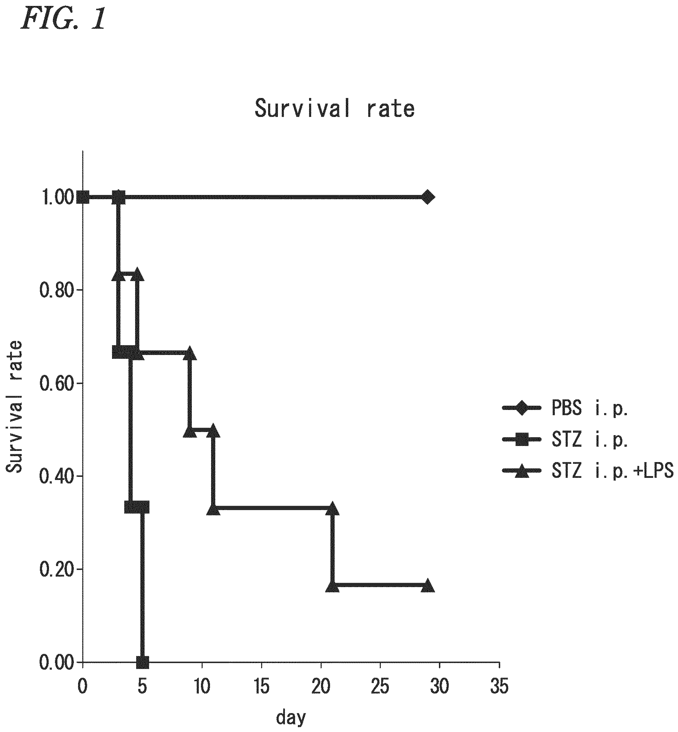

The chemotherapeutic agent STZ is known to produce various toxic effects (nephropathy, myelosuppression (leukocytopenia, lymphocytopenia, neutropenia, thrombocytopenia, anemia, etc.), impaired glucose tolerance (hyperglycemia, increased blood insulin, increased insulin C peptide, and positive urine glucose), hepatopathy, etc. (NPL 10). On measuring the survival rate in the present test, it was found that on the 5th day, although there were no dead mice in the PBS administration group (PBS i.p.), 6 out of 6 mice died in the STZ administration group (STZ i.p.), and 4 out of 6 mice survived in the STZ + LPS administration group (STZ i.p.+LPS). On the 29th day, 1 out of 6 mice survived in the STZ + LPS administration group (Kaplan-Meier test: P<0.05). From the above, it was shown that LPSp administration has an effect of reducing the toxic effects of STZ (FIG. 1, Table 1).

**[Table 1]**

| Survival rate (%) | | | |
|---|---|---|---|
| day | PBS i.p. | STZ i.p. | STZ i.p.+LPS |
| 3 | 100 | 66.7 | 83.3 |
| 4 | 100 | 33.3 | 83.3 |
| 4.5 | 100 | 33.3 | 66.7 |
| 5 | 100 | 0.0 | 66.7 |
| 9 | 100 | 0.0 | 50.0 |
| 11 | 100 | 0.0 | 33.3 |
| 21 | 100 | 0.0 | 16.7 |
| 29 | 100 | 0.0 | 16.7 |

Although prevention of side effects of a cancer chemotherapeutic agent by LPS oral administration was examined with Pantoea agglomerans-derived LPS (LPSp), this effect is obtained with all LPSs that can induce a protective action to tissue damage by activation of macrophages and is thus not limited to LPS derived from bacteria belonging to genus Pantoea.

### [Example 2]

Reduction of toxic effects of the platinating agent cisplatin by LPS oral administration
(1) Method
   1) Reagents
      - Pantoea agglomerans-derived LPS (LPSp) (mac0001 of Macrophi Inc.)
      - Cisplatin (CDDP) (033-20091 of FUJIFILM Wako Pure Chemical Corporation)
   2) Mice and husbandry
      Four-week-old male ICR mice were used. Husbandry of the mice was carried out in a temperature and humidity controlled animal facility under environmental conditions of ad libitum food and water, and 12 hr light/12 hr dark. As food, CE-12 of CLEA Japan, Inc. was provided. After measuring the body weights at the time of delivery, the mice were divided into 3 groups of 6 mice each.
   3) Test

Physiological saline administration group (saline i.p.): 0.5 mL of physiological saline were injected into the peritoneal cavity of each mouse.

Cisplatin administration group (CDDP i.p.): A cisplatin solution (17.4 mg/kg) was injected into the peritoneal cavity of each mouse.

Cisplatin + LPS administration group (CDDP i.p.+LPS): The cisplatin solution (17.4 mg/kg) was injected into the peritoneal cavity of each mouse and LPSp was mixed in drinking water such as to be of 1 mg/kg body weight/day and allowed to be ingested ad libitum.

### (2) Results

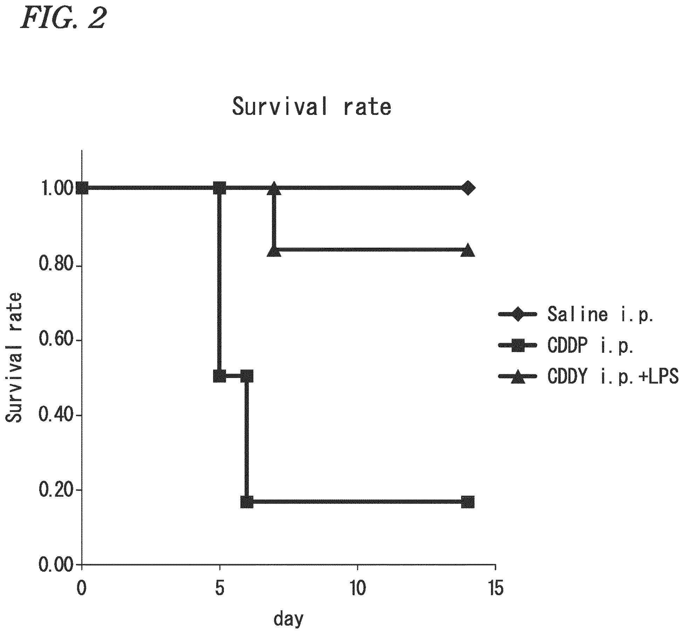

The chemotherapeutic agent cisplatin is known for various side effects (acute renal failure, abnormal BUN value, hematuria, etc.). On measuring the survival rate in the present test, it was found that by the 6th day from anticancer agent administration, although there were no dead mice in the physiological saline administration group (saline i.p.) and the cisplatin + LPS administration group (CDDP i.p.+LPS), 5 out of 6 mice died in the cisplatin administration group (CDDP i.p.). On the 14th day, 1 out of 6 mice survived in the cisplatin administration group and 5 out of 6 mice survived in the cisplatin + LPS administration group (Kaplan-Meier test: P<0.05). From the above, it was shown that LPSp administration has an effect of reducing the toxic effects of cisplatin (FIG. 2, Table 2).

**[Table 2]**

| Survival rate (%) | | | |
|---|---|---|---|
| day | Saline i.p. | CDDP i.p. | CDDP i.p.+LPS |
| 5 | 100 | 50 | 100 |
| 6 | 100 | 16.7 | 100 |
| 7 | 100 | 16.7 | 83.3 |
| 14 | 100 | 16.7 | 83.3 |

### [Example 3]

Reduction of toxic effects of the alkylating agent cyclophosphamide by LPS oral administration
(1) Method
   1) Reagents
      - Pantoea agglomerans-derived LPS (LPSp) (mac0001 of Macrophi Inc.)
      - Cyclophosphamide (CY) (030-12953 of FUJIFILM Wako Pure Chemical Corporation)
   2) Mice and husbandry
      Four-week-old male ICR mice were used. Husbandry of the mice was carried out in a temperature and humidity controlled animal facility under environmental conditions of ad libitum food and water, and 12 hr light/12 hr dark. As food, CE-12 of CLEA Japan, Inc. was provided. After measuring the body weights at the time of delivery, the mice were divided into 3 groups of 6 mice each.
   3) Test

Physiological saline administration group (saline i.p.): 0.5 mL of physiological saline were injected into the peritoneal cavity of each mouse.

Cyclophosphamide administration group (CY i.p.): A cyclophosphamide solution (440 mg/kg) was injected into the peritoneal cavity of each mouse.

Cyclophosphamide + LPS administration group (CY i.p.+LPS): The cyclophosphamide solution (440 mg/kg) was injected into the peritoneal cavity of each mouse and LPSp was mixed in drinking water such as to be of 1 mg/kg body weight/day and allowed to be ingested ad libitum.

### (2) Results

The chemotherapeutic agent cyclophosphamide is known for various side effects (leukocytopenia, vomiting, hair loss, etc.). On measuring the body weights in the present test, significant weight loss in comparison to the physiological saline administration group (saline i.p.) was seen with the cyclophosphamide administration group (CY i.p.) and the cyclophosphamide + LPS administration group (CY i.p.+LPS) on the 3rd day from anticancer agent administration. On the 11th day and 13th day from administration, although significant weight loss in comparison to the physiological saline administration group was seen with the cyclophosphamide administration group (Tukey's multiple comparison test: P<0.05), a significant difference was not seen with the cyclophosphamide + LPS administration group (Table 3). From the above, it was shown that LPSp administration has an effect of suppressing weight loss due to cyclophosphamide.

**[Table 3]**

| Mice body weights after anticancer agent administration (relative value in % with body weights for saline i.p. being 100) | | | | |
|---|---|---|---|---|
| | 0 day | 3 day | 11 day | 13 day |
| Saline i.p. | 100±2.9 | 100±3.3 | 100±3.0 | 100±2.4 |
| CY i.p. | 98.6±3.0 | 80.3±4.4 * * | 85.1±3.2 * | 84.8±3.9 * |
| CY i.p. +LPS | 100±8. 9 | 82.9113.3 ** | 91.8±13.1 | 92.3±13.5 |

| | | | | |
|---|---|---|---|---|
| *: P<0.05; **: P<0.01 (with respect to saline i.p.) | | | | |

### [Example 4]

Reduction of toxic effects of the antimetabolite 5-fluorouracil by LPS oral administration
(1) Method
   1) Reagents
      - Pantoea agglomerans-derived LPS (LPSp) (mac0001 of Macrophi Inc.)
      - 5-fluorouracil (5-FU) (068-01401 of FUJIFILM Wako Pure Chemical Corporation)
   2) Mice and husbandry
      Four-week-old male ICR mice were used. Husbandry of the mice was carried out in a temperature and humidity controlled animal facility under environmental conditions of ad libitum food and water, and 12 hr light/12 hr dark. As food, CE-12 of CLEA Japan, Inc. was provided. After measuring the body weights at the time of delivery, the mice were divided into 3 groups of 6 mice each.
   3) Test

Physiological saline administration group (saline i.p.): 0.5 mL of physiological saline were injected into the peritoneal cavity of each mouse.

5-fluorouracil administration group (5-FU i.p.): A 5-fluorouracil solution (360 mg/kg) was injected into the peritoneal cavity of each mouse.

5-fluorouracil + LPS administration group (5-FU i.p.+LPS): The 5-fluorouracil solution (360 mg/kg) was injected into the peritoneal cavity of each mouse and LPSp was mixed in drinking water such as to be of 1 mg/kg body weight/day and allowed to be ingested ad libitum.

### (2) Results

The chemotherapeutic agent 5-fluorouracil is known for various side effects (leukocytopenia, diarrhea, melena, dysorexia, etc.). On measuring the body weights in the present test, on the 3rd day from anticancer agent administration, whereas a comparison of the physiological saline administration group (saline i.p.) and the 5-fluorouracil administration group (5-FU i.p.) showed a significant weight loss in the 5-fluorouracil administration group (5-FU i.p.) (Tukey's multiple comparison test: P<0.05), a significant difference was not seen between the physiological saline administration group (saline i.p.) and the 5-fluorouracil + LPS administration group (5-FU i.p.+LPS).

Also, on the 6th day, a comparison of the 5-fluorouracil administration group (5-FU i.p.) and the 5-fluorouracil + LPS administration group (5-FU i.p.+LPS) showed the 5-fluorouracil administration group (5-FU i.p.) to be significantly low (Tukey's multiple comparison test: P<0.05). From the above, it was shown that LPSp administration has an effect of reducing the toxic effects of 5-fluorouracil (Table 4).

**[Table 4]**

| Mice body weights after anticancer agent administration (relative value in % with body weights for saline i.p. being 100) | | | |
|---|---|---|---|
| | 0 day | 3 day | 6 day |
| Saline i.p. | 100±5.7 | 100±6.2 | 100±10.7 |
| 5-FU i.p. | 102.0±2.4 | 91.8±4.1 * | 94.5±2.6 |
| 5-FU i.p.+LPS | 106.1±5.7 | 98.1±5.6 | 106.4±5.9 ** |

| | | | |
|---|---|---|---|
| *: P<0.05 (with respect to saline i.p.) **: P<0.05 (with respect to 5-FU i.p.) | | | |

### [Example 5]

Reduction of toxic effects of the alkylating agent streptozotocin by LPS oral administration
(1) Method
   1) Reagents
      - LPS (LPSea) derived from Enterobacter asburiae belonging to genus Enterobacter of family Enterobacteriaceae (Macrophi Inc.)
      - Streptozotocin (STZ) (SAJ-S0130 of Sigma-Aldrich)
   2) Mice and husbandry
      Six-week-old male C57BL mice were used. Husbandry of the mice was carried out in a temperature and humidity controlled animal facility under environmental conditions of ad libitum food and water, and 12 hr light/12 hr dark. As food, the D12450B diet of Research Diets, Inc. was provided. After measuring the body weights at the time of delivery, the mice were divided into 3 groups of 6 mice each.
   3) Test

Physiological saline administration group (saline i.p.): 0.5 mL of physiological saline were injected into the peritoneal cavity of each mouse.

STZ administration group (STZ i.p.) : An STZ solution (313 mg/kg) was injected into the peritoneal cavity of each mouse.

STZ + LPS administration group (STZ i.p.+LPSea): The cisplatin solution (313 mg/kg) was injected into the peritoneal cavity of each mouse and LPSea was mixed in drinking water such as to be of 1 mg/kg body weight/day and allowed to be ingested ad libitum.

### (2) Results

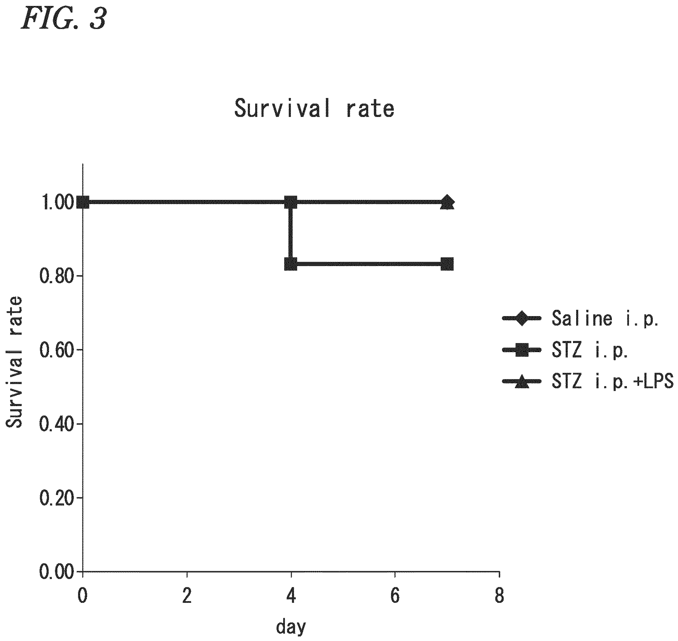

On measuring the survival rate in the present test, it was found that by the 4th day from anticancer agent administration, although there were no dead mice in the physiological saline administration group (saline i.p.) and the STZ + LPS administration group (STZ i.p.+LPSea), 1 out of 6 mice died in the STZ administration group (STZ i.p.). From the above, it was shown that LPSea administration has an effect of reducing the toxic effects of streptozotocin (FIG. 3, Table 5).

**[Table 5]**

| Survival rate (%) | | | |
|---|---|---|---|
| day | Saline i.p. | STZ i.p. | STZ i.p.+LPSea |
| 3 | 100 | 100 | 100 |
| 4 | 100 | 83.33 | 100 |
| 8 | 100 | 83.33 | 100 |

In NPL 11, it is stated that in comparison to using the anticancer agent doxorubicin alone, there is an increase in IFN-γ etc., that contribute to cancer treatment when IP-PA1, which is a Pantoea agglomerans-derived LPS, is used in combination, that is, cancer treatment effects are improved by combined use of Pantoea agglomerans-derived LPS with doxorubicin.

On the other hand, the present invention suppresses the side effects of anticancer agents and can therefore improve the quality of medical treatment life to provide opportunities for treatment by anticancer agents to a larger number of patients and further enables larger amounts of anticancer agents to be administered to patients.

## Claims

1. A cancer chemotherapy supporting agent, food, or drug for use in reducing side effects of cancer chemotherapy and increasing the administration amounts of chemotherapeutic agents and containing a lipopolysaccharide as an active ingredient,
wherein the supporting agent, food, or drug is orally administered,
wherein the cancer chemotherapy is that in which an alkylating agent is administered, wherein the alkylating agent is streptozotocin, and the side effect is a decreased survival rate induced by streptozotocin.

2. A cancer chemotherapy supporting agent, food, or drug for use in reducing side effects of cancer chemotherapy and increasing the administration amounts of chemotherapeutic agents and containing a lipopolysaccharide as an active ingredient,
wherein the supporting agent, food, or drug is orally administered,
wherein the cancer chemotherapy is that in which a platinating agent is administered, wherein the platinating agent is cisplatin, and the side effect is a decreased survival rate induced by cisplatin.

3. A cancer chemotherapy supporting agent, food, or drug for use in reducing side effects of cancer chemotherapy and increasing the administration amounts of chemotherapeutic agents and containing a lipopolysaccharide as an active ingredient,
wherein the supporting agent, food, or drug is orally administered,
wherein the cancer chemotherapy is that in which an alkylating agent is administered, wherein the alkylating agent is cyclophosphamide, and the side effect is weight loss induced by cyclophosphamide.

4. A cancer chemotherapy supporting agent, food, or drug for use in reducing side effects of cancer chemotherapy and increasing the administration amounts of chemotherapeutic agents and containing a lipopolysaccharide as an active ingredient,
wherein the supporting agent, food, or drug is orally administered,
wherein the cancer chemotherapy is that in which an antimetabolite is administered, wherein the antimetabolite is 5-fluorouracil, and the side effect is weight loss induced by 5-fluorouracil.

5. The cancer chemotherapy supporting agent, food, or drug for use according to any one of Claims 1 to 4, wherein the lipopolysaccharide is derived from a bacterium belonging to family Enterobacteriaceae.

6. The cancer chemotherapy supporting agent, food, or drug for use according to any one of Claims 1 to 4, wherein the lipopolysaccharide is derived from a bacterium belonging to genus Pantoea or derived from a bacterium belonging to genus Enterobacter.

## Patentansprüche

1. Krebschemotherapie unterstützendes Mittel, Nahrungsmittel oder Arzneimittel, zur Verwendung beim Verringern von Nebenwirkungen einer Krebschemotherapie und beim Erhöhen der Verabreichungsmengen von chemotherapeutischen Mitteln, und ein Lipopolysaccharid als einen Wirkstoff enthaltend,
wobei das unterstützende Mittel, Nahrungsmittel oder Arzneimittel oral verabreicht wird,
wobei die Krebschemotherapie diejenige ist, bei der ein Alkylierungsmittel verabreicht wird, wobei das Alkylierungsmittel Streptozotocin ist, und die Nebenwirkung eine durch Streptozotocin induzierte verringerte Überlebensrate ist.

2. Krebschemotherapie unterstützendes Mittel, Nahrungsmittel oder Arzneimittel, zur Verwendung beim Verringern von Nebenwirkungen einer Krebschemotherapie und beim Erhöhen der Verabreichungsmengen von chemotherapeutischen Mitteln, und ein Lipopolysaccharid als einen Wirkstoff enthaltend,
wobei das unterstützende Mittel, Nahrungsmittel oder Arzneimittel oral verabreicht wird,
wobei die Krebschemotherapie diejenige ist, bei der ein Platinierungsmittel verabreicht wird, wobei das Platinierungsmittel Cisplatin ist, und die Nebenwirkung eine durch Cisplatin induzierte verringerte Überlebensrate ist.

3. Krebschemotherapie unterstützendes Mittel, Nahrungsmittel oder Arzneimittel, zur Verwendung beim Verringern von Nebenwirkungen einer Krebschemotherapie und beim Erhöhen der Verabreichungsmengen von chemotherapeutischen Mitteln, und ein Lipopolysaccharid als einen Wirkstoff enthaltend,
wobei das unterstützende Mittel, Nahrungsmittel oder Arzneimittel oral verabreicht wird,
wobei die Krebschemotherapie diejenige ist, bei der ein Alkylierungsmittel verabreicht wird, wobei das Alkylierungsmittel Cyclophosphamid ist, und die Nebenwirkung ein durch Cyclophosphamid induzierter Gewichtsverlust ist.

4. Krebschemotherapie unterstützendes Mittel, Nahrungsmittel oder Arzneimittel, zur Verwendung beim Verringern von Nebenwirkungen einer Krebschemotherapie und beim Erhöhen der Verabreichungsmengen von chemotherapeutischen Mitteln, und ein Lipopolysaccharid als einen Wirkstoff enthaltend,
wobei das unterstützende Mittel, Nahrungsmittel oder Arzneimittel oral verabreicht wird,
wobei die Krebschemotherapie diejenige ist, bei der ein Antimetabolit verabreicht wird, wobei der Antimetabolit 5-Fluorouracil ist, und die Nebenwirkung ein durch 5-Fluorouracil induzierter Gewichtsverlust ist.

5. Krebschemotherapie unterstützendes Mittel, Nahrungsmittel oder Arzneimittel, zur Verwendung nach einem der Ansprüche 1 bis 4, wobei das Lipopolysaccharid von einem Bakterium der Familie Enterobacteriaceae stammt.

6. Krebschemotherapie unterstützendes Mittel, Nahrungsmittel oder Arzneimittel, zur Verwendung nach einem der Ansprüche 1 bis 4, wobei das Lipopolysaccharid von einem Bakterium der Gattung Pantoea stammt oder von einem Bakterium der Gattung Enterobacter stammt.

## Revendications

1. Agent assistant une chimiothérapie anticancéreuse, aliment ou médicament pour une utilisation dans la réduction d'effets secondaires d'une chimiothérapie anticancéreuse et l'augmentation des quantités d'administration d'agents chimiothérapeutiques et contenant un lipopolysaccharide en tant que principe actif,
dans lequel l'agent assistant, l'aliment ou le médicament est administré par voie orale,
dans lequel la chimiothérapie anticancéreuse est celle dans laquelle un agent alkylant est administré, dans lequel l'agent alkylant est de la streptozotocine, et l'effet secondaire est un taux de survie réduit induit par la streptozotocine.

2. Agent assistant une chimiothérapie anticancéreuse, aliment ou médicament pour une utilisation dans la réduction d'effets secondaires d'une chimiothérapie anticancéreuse et l'augmentation des quantités d'administration d'agents chimiothérapeutiques et contenant un lipopolysaccharide en tant que principe actif,
dans lequel l'agent assistant, l'aliment ou le médicament est administré par voie orale,
dans lequel la chimiothérapie anticancéreuse est celle dans laquelle un agent platinant est administré, dans lequel l'agent platinant est du cisplatine, et l'effet secondaire est un taux de survie réduit induit par le cisplatine.

3. Agent assistant une chimiothérapie anticancéreuse, aliment ou médicament pour une utilisation dans la réduction d'effets secondaires d'une chimiothérapie anticancéreuse et l'augmentation des quantités d'administration d'agents chimiothérapeutiques et contenant un lipopolysaccharide en tant que principe actif,
dans lequel l'agent assistant, l'aliment ou le médicament est administré par voie orale,
dans lequel la chimiothérapie anticancéreuse est celle dans laquelle un agent alkylant est administré, dans lequel l'agent alkylant est du cyclophosphamide, et l'effet secondaire est une perte de poids induite par le cyclophosphamide.

4. Agent assistant une chimiothérapie anticancéreuse, aliment ou médicament pour une utilisation dans la réduction d'effets secondaires d'une chimiothérapie anticancéreuse et l'augmentation des quantités d'administration d'agents chimiothérapeutiques et contenant un lipopolysaccharide en tant que principe actif,
dans lequel l'agent assistant, l'aliment ou le médicament est administré par voie orale,
dans lequel la chimiothérapie anticancéreuse est celle dans laquelle an antimétabolite est administré, dans lequel l'antimétabolite est du 5-fluorouracile, et l'effet secondaire est une perte de poids induite par le 5-fluorouracile.

5. Agent assistant une chimiothérapie anticancéreuse, aliment ou médicament pour une utilisation selon l'une quelconque des revendications 1 à 4, dans lequel le lipopolysaccharide est dérivé d'une bactérie appartenant à la famille des Enterobacteriaceae.

6. Agent assistant une chimiothérapie anticancéreuse, aliment ou médicament pour une utilisation selon l'une quelconque des revendications 1 à 4, dans lequel le lipopolysaccharide est dérivé d'une bactérie appartenant au genre Pantoea ou dérivé d'une bactérie appartenant au genre Enterobacter.
